# FASCICULE DE BREVET EUROPEEN

(11) **EP 1 585 534 B1**
(45) Date de publication et mention de la délivrance du brevet: **11.04.2007**
(21) Numéro de dépôt: 04704636.2
(22) Date de dépôt: 23.01.2004
(51) Int. Cl.: A61K 35/74, A61K 8/99, A61Q 19/00

(54) **COMPOSITION COMPRENANT UN EXTRAIT D APHANIZOMENON FLOS-AQUAE, SON UTILISATION ET SA PREPARATION**
ZUSAMMENSETZUNG MIT EINEM EXTRAKT AUS APHANIZOMENON FLOS-AQUAE, SEINE VERWENDUNG UND ZUBEREITUNG
COMPOSITION COMPRISING AN EXTRACT OF APHANIZOMENON FLOS-AQUAE, USE THEREOF AND PREPARATION OF SAME

(30) Priorité: 24.01.2003 FR 0300818
(43) Date de publication de la demande: 19.10.2005
(73) Titulaire: Siricie, 78160 Marly le Roi (FR)
(72) Inventeur: JANAILHAC, Marie-Claire, F-75006 Paris (FR); RENARD, Catherine, F-75009 Paris (FR)
(74) Mandataire: de Saint-Palais, Arnaud Marie
(86) Numéro de dépôt international: PCT/FR2004/000167
(87) Numéro de publication internationale: WO 2004/066969

(56) Documents cités:
- PATENT ABSTRACTS OF JAPAN vol. 2002, no. 07, 3 juillet 2002 (2002-07-03) & JP 2002 069443 A (MICROALGAE CORPORATION), 8 mars 2002 (2002-03-08)
- DATABASE BIOSIS [Online] BIOSCIENCES INFORMATION SERVICE, PHILADELPHIA, PA, US; novembre 2001 (2001-11), PUGH N ET AL: "Characterization of human monocyte activation by a water soluble preparation of Aphanizomenon flos-aquae." XP002254817 Database accession no. PREV200200205541 & PHYTOMEDICINE (JENA), vol. 8, no. 6, novembre 2001 (2001-11), pages 445-453, ISSN: 0944-7113

## Description

La présente invention concerne une composition comprenant un extrait d'algue *Aphanizomenon flos-aquae* applicable par voie topique. Elle s'applique plus particulièrement, mais non exclusivement, au traitement des couches supérieures de l'épiderme et/ou du cheveu, notamment à la prévention et au traitement du vieillissement cutané induit et/ou à la réparation de certaines altérations des tissus cutanés telles que les vergetures et/ou à la contribution à l'embellissement du cheveu.

De façon générale, le vieillissement cutané induit est provoqué par des facteurs extrinsèques (vieillissement photo-induit et environnemental). Au niveau de la peau, l'exposition environnementale quelle soit solaire ou quelle soit due aux polluants atmosphériques se traduit par des ridules et rides profondes, des télangiectasies et des lésions purpuriques, des tâches pigmentaires et hyperplasie sébacée, des troubles de l'hydratation cutanée, une augmentation de la perte transépidermique en eau, une modification des lipides de surface, une augmentation de la desquamation cutanée.

Les plus importantes modifications histologiques liées au vieillissement induit siègent au niveau du derme, notamment des fibroblastes, des composants de la matrice extracellulaire et du réseau vasculaire. La jonction dermo-épidermique s'affaisse, entraînant une diminution des points d'attache entre le derme et l'épiderme. Une altération simultanée des cellules de l'épiderme est également notée avec une perte de polarité des kératinocytes, une diminution du nombre de cellules de Langherans etc....

La présente invention concerne l'utilisation d'une variété unique de cyanobactéries, variété découverte dans le lac Klamath, Oregon (USA) et
caractérisée par Renhui et *al*. (Renhui Li, Wayne W. Carmichael, Yongding Liu & Makoto M. Watanabe, Hydrobiology, 438: 99-105, 2000, Taxonomic reevaluation of *Aphanizomenon-flos-aquae* NH-5 based on morphology and 16S rRNA gène sequences).
Les cyanobactéries constituent un groupe particulier de procaryotes autrefois rattaché au règne végétal. Ce sont des êtres vivants unicellulaires très petits souvent bleu-vert (d'où le nom d'algues bleues) autotrophes et d'une organisation relativement simple. Elles ont l'aptitude de croître dans des milieux extrêmes et de fixer le diazote.

Des préparations à base d'*Aphanizomenon flos-aquae* séchées sont préconisées en tant que complément alimentaire pour leurs nombreux constituants, notamment leur forte teneur en protéines hautement assimilables et la présence des vitamines B6, B12 et F.

En effet, les études répertoriées montrent que l'administration orale d'*Aphanizomenon flos-aquae:*
- permet d'augmenter la réactivité du système immunitaire par augmentation de la synthèse d'ARN messager codant pour l'Interleukine 1 (IL-1) (Characterization of human monocyte activation by a water soluble préparation of *Aphanizomenon flos-aquae,* Phytomedicine, Pugh N, Pasco DS, 2001, Nov 8(6) : 445-53),
- est bénéfique pour la santé grâce à la diversité des nutriments qui la compose (Microalgae as food & supplément, Kay RA, Crit. Rev. Food Sci. Nut., 1991, 30(6) : 555-73),
- est une bonne source nutritionnelle en acides gras polyinsaturés qui lui confèrent une propriété hypocholestérolémiante (Rafail 1. Kushak, Christian Drapeau, Elisabeth M.Van Cott, Harland H; Winter, JANA, Vol.2 (3), 2000, 59-65).

En revanche, aucun document ne fait référence à l'utilisation de l'*Aphanizomenon tlos-aquae* pour la préparation de compositions bénéfiques pour la prévention du vieillissement cutané et l'embellissement des cheveux notamment en vue d'une application topique.

Or, une incorporation *per se* d'*Aphanizomenon flos-aquae* dans des compositions utilisables par voie topique n'est pas possible compte tenu du faible degré de solubilisation de l'algue séchée, de sa forte coloration, de sa forte odeur et du manque de stabilité de ses composés biochimiques.

L'invention a donc pour objet de résoudre ces inconvénients par la mise au point d'une composition applicable par voie topique, qui permet de conserver les principes actifs de l'*Aphanizomenon flos-aquae* dans toute leur intégrité afin de participer activement au traitement des couches supérieures de l'épiderme et/ou du cheveu notamment à la prévention du vieillissement cutané et à l'embellissement du cheveu.

A cet effet, elle propose une composition applicable par voie topique comportant au moins un extrait d'*Aphanizomenon flos-aquae* à une concentration comprise entre 0,01 et 10% en matière sèche du poids total de la composition.

Avantageusement, un procédé pour la préparation de ladite composition comportant au moins un extrait d'*Aphanizomenon flos-aquae* comprend la préparation dudit extrait par extraction des substances actives contenues dans l'*Aphanizomenon flos-aquae* par exemple sèche lyophilisée notamment selon les étapes suivantes :
- au moins une macération à une température de 25 à 50°C d'algues bleues séchées de l'espèce *Aphanizomenon flos-aquae* en présence d'enzymes telles que des cellulases, des pectinases et des glucanases pendant un temps de dix minutes à dix heures sous agitation,
- une séparation liquide/solide par centrifugation,
- une séparation liquide/liquide par un procédé de filtration membranaire,
- un séchage et/ou une dilution dans une solution contenant des adjuvants spécifiques, par exemple du sorbitol,
- une éventuelle séparation spécifique des divers constituants ainsi extraits, par exemple par chromatographie, les différentes substances obtenues pouvant être utilisées seules ou en mélange, selon l'effet recherché.

L'étape de séchage pourra aussi bien être un séchage classique (chaleur) qu'un séchage par nébulisation ou lyophilisation.

Par ce procédé, les substances ayant une activité majeure sur la peau et le cheveu sont extraites, à savoir :
- des caroténoïdes,
- des phycocyanines,
- des acides aminés, notamment la méthionine, la lysine, la proline et la serine,
- des polysaccharides.

Le susdit extrait pourra être dissous dans une solution aqueuse telle qu'un mélange eau/sorbitol.

Ladite composition pourra se présenter sous la forme d'émulsions simples ou multiples telles qu'une émulsion eau/huile ou huile/eau ou encore une émulsion triphasique et/ou un gel ou une solution aqueuse ou hydroalcoolique.

Ladite composition pourra également se présenter sous la forme d'un système vectorisé à libération contrôlée ou à libération modulée.

Des modes d'exécution de l'invention et des exemples de formulations dudit cosmétique et/ou composition dermatologique seront décrits, ci-après, à titre d'exemples non limitatifs.

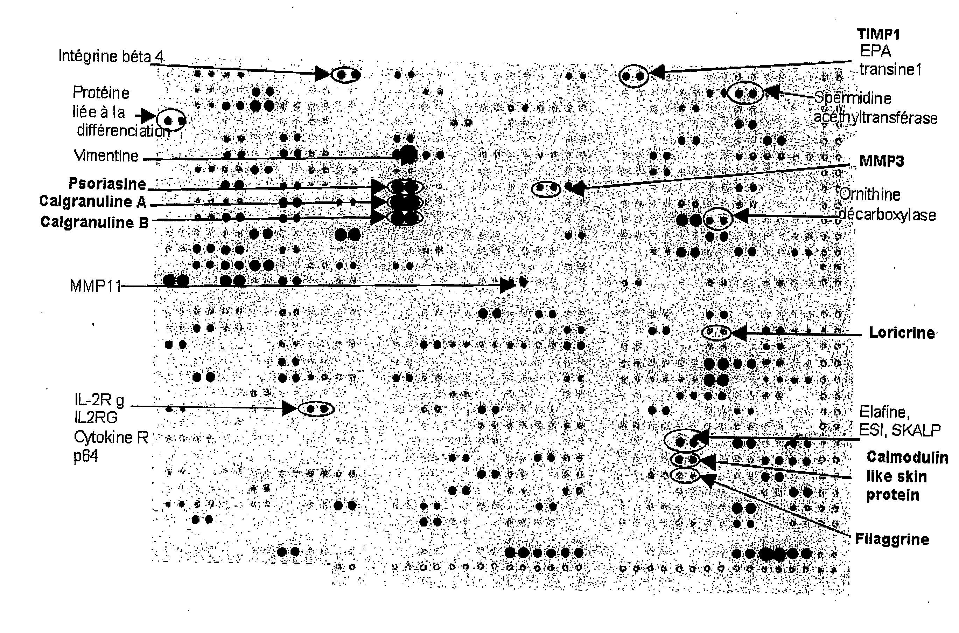

La figure unique est la représentation d'un schéma d'un profil obtenu par hybridation de sondes d'ADN complémentaires marquées avec les différents ARNm obtenus avec un épiderme normal humain traité avec un extrait brut aqueux d'*Aphanizomenon flos-aquae.*

Le procédé pour la préparation d'une composition comportant au moins un extrait d'*Aphanizomenon flos-aquae* contenant des substances actives comprend la préparation dudit extrait selon les étapes suivantes :
- Au moins une macération à une température de 25 à 50°C et de préférence de 35°C d'algues bleues séchées *Aphanizomenon flos-aquae* en présence de cellulases, de pectinases et de glucanases pendant un temps de dix minutes à dix heures et de préférence de quatre heures sous agitation. Les résultats des essais montrent que l'attaque des différentes enzymes permet une meilleure solubilisation de la paroi parenchymateuse des algues et ainsi une plus grande richesse en polysaccharides de l'extrait aqueux ainsi préparé.
- Une séparation liquide/solide par centrifugation sous une accélération de 5 000 à 10 000g, et de préférence de 9000g.
- Une séparation liquide/liquide par un procédé de filtration membranaire avec un seuil de coupure compris entre 100 000 daltons et 0,2 µm.
- Un séchage et/ou une dilution dans une solution aqueuse de sorbitol. On entend par séchage aussi bien un séchage classique (chaleur) qu'un séchage par nébulisation ou lyophilisation.
- Une séparation spécifique des divers constituants ainsi extraits par chromatographie, les différentes substances obtenues étant utilisées seules ou en mélange, selon l'effet recherché.

L'extrait ainsi obtenu est composé notamment de protéines (entre 0,05 à 1% m/m (rapport massique)), de vitamine B12 (entre 0,003 à 0,05% m/m), de Lysine (entre 0,2 à 3% m/m), de méthionine (entre 0,04 à 0,6% m/m), de proline (entre 0,15 à 2,5% m/m) et de sérine (entre 0,15 à 2,5% m/m).

La méthode des filtres à haute densité ou "cDNA macroarrays" sur un support comprenant au moins 600 gènes caractéristiques du système cutané et du système pileux a été utilisée pour étudier l'effet de l'extrait d'*Aphanizomenon flos-aquae* sur l'expression des gènes codant pour des protéines majeures d'intérêt cosmétique ou dermo-cosmétique.
Dans cette méthode, les dépôts (sondes) sont des clones d'ADNc (acide désoxyribonucléique complémentaire) ou des produits de PCR (« Polymerase Chain Reaction » ou réaction en chaîne de la polymérase), fixés à haute densité, sur une membrane de nylon. Le marquage est le plus souvent radioactif et le criblage est réalisé en excès de cible, on obtient ainsi une mesure de l'abondance relative de chacun des ARNm (acide ribonucléique messager) présents dans l'échantillon de départ.

Les protéines sont obtenues à partir d'explants de peau préparés suite à une plastie mammaire sur un donneur.
Deux échantillons de peau de 20 cm² ont été préparés et maintenus en survie dans un milieu de culture.
L'extrait d'*Aphanizomenon flos-aquae* a été appliqué sur l'explant à raison de 5 mg/cm² d'une solution à 2% d'extrait brut aqueux sans adjuvant le matin et le soir pendant deux jours.
Après chaque application, les explants ont été incubés à 37°C avec 5% de CO₂.
L'explant de peau témoin a été traité selon le même procédé avec de l'eau stérile.
Les morceaux de peau (épidermes) ont été rincés puis ils ont été placés en présence de Tri-reagent ® (Sigma T9424) puis congelés à -80°C.

Les ARN ont été extraits et purifiés à partir des surnageants obtenus après broyage des épidermes congelés.
Les ARN sont traités, d'une part, au moyen d'une enzyme ARNase-out afin d'inhiber les enzymes ARNases.et, d'autre part, au moyen d'une enzyme ADNase 1 pour éliminer les traces d'ADN contaminant l'ARN.

La qualité des ARN est contrôlée par électrophorèse sur gel d'agarose.

Les groupes d'ARNm sont purifiés par hybridation des extrémités poly(A) (chaîne de nucléotides adénosines) des ARNm avec des amorces oligo(dT) (oligonucléotides constitués de desoxythymidines) biotinylées.

Des sondes ADN multiples marquées au phosphore ³³P ont été réalisées par transcription inverse des ARNm liés sur des billes de poly(dT) (chaîne de nucléotides desoxythymidines), à l'aide d'un groupe d'amorces spécifiques des séquences immobilisées sur les filtres en présence de α³³P dATP (α³³P-desoxyadénosine triphosphate).

Les sondes marquées ont été purifiées par chromatographie d'exclusion encore appelée tamisage moléculaire, gel-filtration ou perméation de gel.

La qualité et l'équivalence des sondes marquées ont été évaluées par comptage en scintillation liquide. Les scintillateurs sont des milieux dans lesquels une fraction non négligeable de l'énergie absorbée lors d'une interaction avec une particule α ou β est transformée, par luminescence, en photons susceptibles d'être détectés. Cette détection consiste à les convertir en un signal électrique qui peut être traité par une électronique appropriée.

Des membranes de type "Custom ATLAS BA 600/1" sont prétraitées puis les ADNc immobilisés sur chaque membrane sont hybridés (68°C, 12 heures) avec des sondes marquées correspondantes, les filtres sont ensuite lavés et analysés par quantification directe de la radioactivité des spots à l'aide d'un appareil du type phosphorlmager^{™} (Cyclone, Packard Instrument) et de son logiciel QuantArray^{™} (Packard).

Le tableau I ci-dessous présente les gènes dont l'expression relative (RE) a été significativement modifiée après quarante huit heures d'une application bi-quotidienne d'un extrait brut d'*Aphanizomenon flos-aquae* sur un épiderme normal humain.

**Tableau 1 :**

| | Témoin | Extrait d'*Aphanizomenon-flos-aquae* | |
|---|---|---|---|
| **Nom des gènes** | **RE** | **RE** | **%** |
| Vimentine (VIM) | 10,0 | 21,8 | 217 |
| Métalloprotéase 11 (MMP11) Stromélysine 3 | 6,2 | 15,9 | 255 |
| Métalloprotéase 3 (MMP3); | | | |
| Stromélysine 1 (STMY1; SL1); | | | |
| Transine 1 | 5,7 | 17,3 | 306 |
| Inhibiteur tissulaire de métalloprotéase 1 (TIMP1); | | | |
| Potentiateur de l'activité érythroïde (EPA); | | | |
| Inhibiteur des collagénases fibroblastiques | 10,0 | 24,3 | *244* |
| Sous-unité gamma du récepteur de l'interleukine-2 (IL-2R gamma; IL2RG); | | | |
| Récepteur commun des chaînes gamma des cytokines ; P64 | 6,6 | 20,0 | *302* |
| Filaggrine épidermale (FLG) | 28,7 | 7,3 | 25 |
| Loricrine (LOR; LRN) | 31,9 | 11,7 | 37 |
| Protéine liée à la différenciation des adipocytes | 15,8 | 23,1 | 146 |
| Intégrine bêta 4 (ITGB4); | | | |
| antigène CD104 | 24,7 | 40,1 1 | 162 |
| Protéine liant le calcium S100 A7; Psoriasine | 135,2 | 202,0 | 149 |
| Protéine liant le calcium S100 A8 (S100A8); | | | |
| Calgranuline A (CALA); | | | |
| "migration inhibitory factor-related protein 8" (MRP8); | | | |
| "leukocyte L1 complex light chain; | | | |
| cystic fibrosis antigen" (CFAG) | 311,1 1 | 485,4 | 156 |
| S100 Protéine liant le calcium A9 (S100A9); Calgranuline B (CAGB); | | | |
| "migration inhibitory factor-related protein 14" (MRP14); | | | |
| "leukocyte L1 complex heavy chain"; | 210,2 | 323,8 | 154 |
| Ornithine décarboxylase (ODC) | 9,4 | 18,1 1 | 193 |
| Spermidine acétyltransférase | 13,9 | 26,3 | 189 |
| elafin; Inhibiteur spécifique des élastases (ESI); | | | |
| « skin-derived antileukoproteinase » (SKALP) | 22,6 | 34,3 | 152 |
| « calmodulin-like skin protein » (CLSP) | 31,8 | 16,0 | 50 |

Le schéma de la figure unique représente le profil obtenu par hybridation des sondes d'ADN complémentaires marquées avec les différents ARNm obtenus avec un épiderme normal humain traité avec un extrait brut aqueux d'*Aphanizomenon flos-aquae.*

L'expression des gènes indiqués en gras dans le schéma de la figure unique (calgranuline A, calgranuline B, loricrine, psoriasine, protéine du type calmoduline, MMP3 et TIMP1) a été également quantifiée par la technique de RT-PCR (« Polymerase Chain Reaction Reverse Transcriptase » ou Transcription inverse - Réaction en chaîne de la polymérase) afin de valider les résultats premièrement obtenus.
Dans cette expérience, l'actine a été utilisée comme marqueur de référence.

Les résultats présentés dans le tableau ll ci-dessous ont été obtenus :

| Gène | Expression du gène par rapport au témoin (base 100) - RT-PCR |
|---|---|
| Calgranuline A | 153 |
| Calgranuline B | 166 |
| Filaggrine | 16 |
| Loricrine | 14 |
| Psoriasine | 137 |
| "Calmodulin Like Skin Protein" | 44 |
| Matrice Métalloprotéase 3 | 230 |
| Inhibiteur tissulaire de la Métalloprotéase 1 | 209 |

Le traitement des explants de peau par un extrait d'*Aphanizomenon flos-*aquae induit des modifications significatives dans l'expression de la différenciation et prolifération des cellules de l'épiderme. Ces modifications sont identiques à celles obtenues avec un composé de type rétinol (ou rétinoïde : lipide diffusant directement dans la membrane plasmique) sans que l'extrait *d'Aphanizomenon flos-aquae* n'en présente les contraintes de formulation.

L'expression de la CLSP (« Calmodulin Like Skin Protein » ou protéine de peau du type Calmoduline), un marqueur spécifique de la différenciation des kératinocytes, est considérablement réprimée par des rétinoïdes et leurs analogues dans la stratum granulosum (la troisième couche la plus interne de l'épiderme où la kératine apparaît sous forme de granules) et les couches inférieures du stratum corneum (couche la plus externe de l'épiderme) (Mehul B. et al., Calmodulin-like skin protein: a new marker of keratinocyte differentiation, J. Invest. Dermatol., 2001 Jun, 116(6), 905-9).

L'extrait brut *d'Aphanizomenon flos-aquae* réduit par deux fois l'expression de la CLSP, ceci est un argument en faveur de son implication dans la modulation de ce marqueur.

Par ailleurs, les rétinoïdes inhibent l'expression de la loricrine (Brown L.J. et al., Retinoic acid suppression of loricrin expression in reconstituted human skin cultured at the liquid-air interface, J. Invest. Dermatol., 1994 Jun, 102(6), 886-90), tout comme l'extrait brut *d'Aphanizomenon flos-aquae* qui en réduit de plus de 10 fois l'expression.

Quant aux filaggrines, qui résultent en outre de la digestion des protéines de profilaggrines contenues dans les granules dans la partie inférieure du stratum corneum, elles sont ensuite digérées par des peptidases en acides aminés. L'inhibition des messagers des filaggrines pourrait résulter d'une inhibition globale de la différenciation des kératinocytes et ainsi contribuer à une meilleure cohésion des cornéocytes (dans la couche cornée, le kératinocyte prend le nom de cornéocyte) et donc à une amélioration de la fonction barrière cutanée.

La loricrine est le constituant majeur de la paroi des cornéocytes, et est contenue dans les granules jusqu'au stade terminal de la différenciation et contribue ensuite à la formation de l'enveloppe des cornéocytes afin de la renforcer. La diminution de son expression sous l'effet de l'extrait brut *d'Aphanizomenon flos-aquae* est en cohérence avec l'évolution des expressions des filaggrines et de la CLSP.

En revanche, l'expression des calgranulines A et B, qui sont synthétisées par les cellules épithéliales et les kératinocytes, est augmentée sous l'effet de l'extrait brut *d'Aphanizomenon flos-aquae.* La psoriasine qui, comme la calgranuline A et la calgranuline B, appartient à la famille des protéines S100, et dont l'expression est inductible par les rétinoïdes (Tavakkov A. et al., A retinoic acid-inducible skin-specific gène (RIS-1/psoriasin): molecular cloning and analysis of gene expression in human skin in vivo and cultured skin cells in vitro, Mol. Biol. Rep., 1994, 20(2), 75-83) dans les kératinocytes primaires en différenciation a une expression qui augmente également sous l'effet du traitement. Il en est de même de l'augmentation de l'expression de la MMP3 qui est connue pour être significativement augmentée sous l'effet des rétinoïdes (Varani J. et al., Expression of serine proteases and metalloproteinases in organ-cultured human skin. Altered levels in the présence of retinoic acid and possible relationship to retinoid-induced loss of epidermal cohésion, Am. J. Pathol., 1994, 145, 561-573).

Tous ces événements - activation de l'expression relative des messagers calgranuline A, calgranuline B, psoriasine, métalloprotéase 3 et inhibition de l'expression des messagers filaggrine, loricrine, "calmodulin like skin protein" - laissent entrevoir une action analogue à celle des rétinoïdes (« rétinoïd like ») de l'application topique de l'extrait brut *d'Aphanizomenonflos-aquae.* De plus, l'augmentation de l'expression de l'Inhibiteur Tissulaire de la Métalloprotéase 1 (TIMP1) suppose un effet anti-âge lors de l'application topique d'une composition cosmétique à base *d'Aphanizomenon flos-aquae.*

Une composition pour le soin après soleil comprend :

| | | |
|---|---|---|
| A1* | Eau déminéralisée | qsp** 100% |
| A2 | Sequestrene ® NA4/Celon ® E/Trilon ® B | 0,01% |
| B1 | Nipagin ® M/POB Méthyle | 0,05 % |
| C1 | Carbopol ® 940 | 15,00% |
| D1 | Triéthanolamine | 0,5 à 1 % |
| E1 | Conservateur antimicrobien | 0,5 à 1 % |
| F1 | Silicone | 1 à 2% |
| F2 | Parfum | 0,15% |
| G1 | Extrait *d'Aphanizomenon flos-aquae* | 0,5 à 5% |
| | En solution dans du sorbitol et de l'eau (soit de 1 à 5% d'extrait sec *d'Aphanizomenon flos-aquae*) | |

| | | |
|---|---|---|
| (* : chacune des lettres placées devant un composant représente une phase) (** qsp : quantité suffisante pour) | | |

Une composition pour les soins anti-âge comprend :

| | | |
|---|---|---|
| A1 | Emulium ® (Gattefossé) | 4,0% |
| A2 | Amerchol ® (Amerchol) | 6,0% |
| A3 | Amerlate ® (Amerchol) | 2,0% |
| A4 | Végétol ® huileux calendula (Gattefossé) | 2,0% |
| A5 | LNST ® 98 (Lanatech) | 1,0% |
| B1 | Eau déminéralisée | qsp100% |
| B2 | Carbopol ® (BF Goodrich) | 10,0% |
| C | Abil ® (Goldschmidt) | 6,0% |
| D1 | Eau déminéralisée | 5,0% |
| D2 | Triéthanolamine (Prolabo) | 0,2% |
| E1 | Conservateur antimicrobien | 0,5% |
| E2 | Glycérine naturelle (Elf Atochem) | 4,0% |
| F | Fluidamid ® DF 125 (Roquette) | 4,0% |
| G | Extrait *d'Aphanizomenon flos-aquae* | 0,5 à 5% |
| | En solution dans du sorbitol et de l'eau (soit de 1 à 5% d'extrait sec *d'Aphanizomenon flos-aquae*) | |
| H | Parfum | 0,3% |

Une composition pour le lavage et le soin des cheveux comprend :

| | | |
|---|---|---|
| A1 | Texapon ® (Henkel) | 10,00% |
| B1 | Eau déminéralisée | qsp100% |
| B2 | Sequestrene ® (Prolabo) | 0,05% |
| C1 | Tegobétaïn ® (Goldschmidt) | 10,00% |
| D1 | Empilan ® (Albright & Wilson St Mihiel) | 4,00% |
| E1 | Hydralphatine ® 3P (Lanatech) | 3,00% |
| E2 | Conservateur antimicrobien | 0,50% |
| F1 | Glutamate (Amerchol) | 15,00% |
| G1 | Oramix ® (Seppic) | 6,00% |
| G2 | Simulsol ® (Seppic) | 1,00% |
| H1 | Eau déminéralisée | 5,00% |
| H2 | Acrysol ® (Seppic) | 6,00% |
| J1 | Extrait *d'Aphanizomenon flos-aquae* | 0,5 à 5% |
| | En solution dans du sorbitol et de l'eau (soit de 1 à 5% d'extrait sec *d'Aphanizomenon-flos-aquae*) | |

Une composition soin anti-rides comprend :

| | | |
|---|---|---|
| A1 | Eau déminéralisée | qsp100% |
| A2 | Sepigel ® (Seppic) | 1,0% |
| B1 | Emulium ® (Gattefossé) | 3,0% |
| B2 | Amerchol ® (Amerchol) | 4,0% |
| B3 | Crodamol ® (Croda) | 8,0% |
| B4 | Abil ® (Goldschmidt) | 5,0% |
| C | Conservateur antimicrobien | 0,3% |
| D | Fluidamid ® DF15 (Gattefossé) | 3,0% |
| E | Extrait *d'Aphanizomenon flos-aquae* | 0,5 à 5% |
| | En solution dans du sorbitol et de l'eau (soit de 1 à 5% d'extrait sec *d'Aphanizomenon-flos-aquae*) | |

Une composition masque traitant pour cheveux desséchés comprend :

| | | |
|---|---|---|
| A1 | Cetaryl glucoside (Montanov ® 68 - SEPPIC) | 7 % |
| A2 | Coco betaïne (AMONYL ® 265 BA - SEPPIC) | 0,5 % |
| A3 | Beurre de karité | 4 % |
| A4 | Cire d'abeille | 2 % |
| A5 | Dimethicone (DOW CORNING) | 5 % |
| B1 | Eau déminéralisée | qsp 100% |
| B2 | Decyl glucoside (ORAMIX ® NS 10 - SEPPIC) | 1 % |
| C1 | Parfum | 0,5% |
| C2 | Conservateur antimicrobien | 0,5% |
| C3 | Extrait *d'Aphanizomenon flos-aquae* | 0,5 à 5% |
| | En Solution dans du sorbitol et de l'eau (soit de 1 à 5% d'extrait sec *d'Aphanizomenon flos-aquae*) | |

Une crème de nuit comprend :

| | | |
|---|---|---|
| A1 | Cetearyl glucoside (Montanov ® 68 - SEPPIC) | 6 % |
| A2 | Huiles végétales | 20 % |
| A3 | DL-alpha-tocopherol (BASF) | 0,05% |
| B1 | Eau déminéralisée | qsp 100% |
| C1 | Conservateur antibactérien | 0,5% |
| C2 | Parfum | 0,3 % |
| C4 | Extrait *d'Aphanizomenon flos-aquae* | 0,5 à 5% |
| | En Solution dans du sorbitol et de l'eau (soit de 1 à 5% d'extrait sec *d'Aphanizomenon flos-aquae*) | |

## Revendications

1. Composition applicable par voie topique,
**caractérisée en ce qu'**elle comporte au moins un extrait *d'Aphanizomenon flos-aquae* à une concentration comprise entre 0,01 et 10% en matière sèche du poids total de la composition.

2. Composition selon la revendication 1,
**caractérisée en ce qu'**elle se présente sous la forme d'émulsions simples ou multiples telles qu'une émulsion eau/huile ou huile/eau ou encore une émulsion triphasique et/ou un gel ou une solution aqueuse ou hydroalcoolique.

3. Composition selon la revendication 1,
**caractérisée en ce qu'**elle se présente sous la forme d'un système vectorisé à libération contrôlée ou à libération modulée.

4. Utilisation d'une composition selon la revendication 1 pour la fabrication de produits pour le traitement des couches supérieures de l'épiderme et/ou du cheveu.

5. Utilisation d'une composition selon l'une des revendications 1 à 4 pour la fabrication d'un cosmétique.

6. Utilisation d'une composition selon l'une des revendications 1 à 4 pour la fabrication d'une composition dermatologique.

7. Composition pour le soin après soleil,
**caractérisée en ce qu'**elle comprend :
■ Eau déminéralisée qsp* 100%
■ Sequestrene ® NA4/Celon ® E/Trilon ® B 0,01 %
■ Nipagin ® M/POB Méthyle 0,05 %
■ Carbopol ® 940 15,00%
■ Triéthanolamine 0,5 à 1 %
■ Conservateur antimicrobien 0,5 à 1 %
■ Silicone 1 à 2%
■ Parfum 0,15%
■ Extrait *d'Aphanizomenon flos-aquae*
En solution dans du sorbitol et de l'eau (soit de 1 à 5% d'extrait sec *d'Aphanizomenon flos-aquae)* 0,5 à 5%

8. Composition pour les soins anti-âge,
**caractérisée en ce qu'**elle comprend :
■ Emulium ® 4,0%
■ Amerchol ® 6,0%
■ Amerlate ® 2,0%
■ Végétol ® huileux calendula 2,0%
■ LNST ® 98 1,0%
■ Eau déminéralisée qsp100%
■ Carbopol ® 10,0%
■ Abil ® 6,0%
■ Eau déminéralisée 5,0%
■ Triéthanolamine 0,2%
■ Conservateur antimicrobien 0,5%
■ Glycérine naturelle 4,0%
■ Fluidamid ® DF 125 4,0%
■ Extrait *d'Aphanizomenon flos-aquae*
En solution dans du sorbitol et de l'eau (soit de 1 à 5% d'extrait sec *d'Aphanizomenon flos-aquae)* 0,5 à 5%
■ Parfum 0,3%

9. Composition pour le lavage et le soin des cheveux,
**caractérisée en ce qu'**elle comprend :
■ Texapon ® 10,00%
■ Eau déminéralisée qsp100%
■ Sequestrene ® 0,05%
■ Tegobétaïn ® 10,00%
■ Empilan ® 4,00%
■ Hydralphatine 3P 3,00%
■ Conservateur antimicrobien 0,50%
■ Glutamate 15,00%
■ Oramix ® 6,00%
■ Simulsol ® 1,00%
■ Eau déminéralisée 5,00%
■ Acrysol ® 6,00%
■ Extrait *d'Aphanizomenon flos-aquae*
En solution dans du sorbitol et de l'eau (soit de 1 à 5% d'extrait sec *d'Aphanizomenon flos-aquae)* 0,5 à 5%

10. Composition soin anti-rides,
**caractérisée en ce qu'**elle comprend :
■ Eau déminéralisée qsp100%
■ Sepigel ® 1,0%
■ Emulium ® 3,0%
■ Amerchol ® 4,0%
■ Crodamol ® 8,0%
■ Abil ® 5,0%
■ Conservateur antimicrobien 0,3%
■ Fluidamid ® DF15 3,0%
■ Extrait *d'Aphanizomenon flos-aquae*
En solution dans du sorbitol et de l'eau (soit de 1 à 5% d'extrait sec *d'Aphanizomenon flos-aquae)* 0,5 à 5%

11. Composition masque traitant pour cheveux desséchés,
**caractérisée en ce qu'**elle comprend :
■ Cetaryl glucoside 7 %
■ Coco betaïne 0,5 %
■ Beurre de karité 4 %
■ Cire d'abeille 2 %
■ Dimethicone 5 %
■ Eau déminéralisée qsp 100%
■ Decyl glucoside 1 %
■ Parfum 0,5%
■ Conservateur antimicrobien 0,5%
■ Extrait *d'Aphanizomenon flos-aquae*
En Solution dans du sorbitol et de l'eau (soit de 1 à 5% d'extrait sec *d'Aphanizomenon flos-aquae)* 0,5 à 5%

12. Crème de nuit,
**caractérisée en ce qu'**elle comprend :
■ Cetearyl glucoside 6 %
■ Huiles végétales 20 %
■ DL-alpha-tocopherol 0,05%
■ Eau déminéralisée qsp 100%
■ Conservateur antibactérien 0,5%
■ Parfum 0,3 %
■ Extrait d'Aphanizomenon flos-aquae
En Solution dans du sorbitol et de l'eau (soit de 1 à 5% d'extrait sec d'Aphanizomenon flos-aquae) 0,5 à 5%

13. Procédé pour la préparation d'une composition selon la revendication 1,
**caractérisé en ce qu'**il comprend les étapes suivantes :
- au moins une macération à une température de 25 à 50°C et, de préférence, 35°C d'algues bleues séchées d'*Aphanizomenon flos-aquae* en présence d'enzymes telles que des cellulases, des pectinases et des glucanases pendant un temps de dix minutes à dix heures et de préférence quatre heures sous agitation,
- une séparation liquide/solide par centrifugation,
- une séparation liquide/liquide par un procédé de filtration membranaire,
- un séchage et/ou une dilution dans une solution contenant des adjuvants spécifiques, par exemple du sorbitol,
- une éventuelle séparation spécifique des divers constituants ainsi extraits, par exemple par chromatographie, les différentes substances obtenues pouvant être utilisées seules ou en mélange.

14. Procédé selon la revendication 13,
**caractérisé en ce que** l'étape de séchage est un séchage classique (chaleur) ou un séchage par nébulisation ou lyophilisation.

15. Procédé selon la revendication 13,
**caractérisé en ce que** le susdit extrait est dissous dans une solution aqueuse telle qu'un mélange eau/sorbitol.

## Claims

1. A topically applicable composition,
**characterized in that** it comprises at least one *Aphanizomenon flos-aquae* extract at a concentration between 0.01 and 10% by dry weight of the total weight of the composition.

2. The composition according to claim 1,
**characterized in that** it exists in the form of simple or multiple emulsions such as a water/oil emulsion or an oil/water emulsion or even a triphasic emulsion, and/or a gel, or an aqueous or hydroalcoholic solution.

3. The composition according to claim 1,
**characterized in that** it exists in the form of a vectorized system with controlled release or modulated release.

4. The use of a composition according to claim 1, in order to make products for treating upper layers of the epidermis and/or hair.

5. The use of a composition according to any of claims 1 to 4, for making a cosmetic.

6. The use of a composition according to any of claims 1 to 4, for making a dermatological composition.

7. A composition for after-sun care, **characterized in that** it comprises:
■ Demineralized water qs*100%
■ Sequestrene®NA4/Celon®E/Trilon®B 0.01 %
■ Nipagin®M/methyl-POB 0.05%
■ Carbopol ®940 15.00%
■ Triethanolamine 0.5-1 %
■ Antimicrobial preservative 0.5-1%
■ Silicone 1-2%
■ Perfume 0.15%
■ *Aphanizomenon flos-aquae* extract
in solution in sorbitol and water
(i.e. 1-5% of dry *Aphanizomenon flos-aquae* extract) 0.5-5%

8. A composition for after-sun care, **characterized in that** it comprises:
■ Emulium ® 4.0%
■ Amerchol® 6.0%
■ Amerlate ® 2.0%
■ Oily calendula Vegetol ® 2.0%
■ LNST ® 98 1.0%
■ Demineralized water qs100%
■ Carbopol ® 10.0%
■ Abil ® 6.0%
■ Demineralized water 5.0%
■ Triethanolamine .2%
■ Antimicrobial preservative 0.5%
■ Natural glycerin 4.0%
■ Fluidamid ®DF 125 4.0%
■ *Aphanizomenon flos-aquae* extract
in solution in sorbitol and water
(i.e. 1-5% of dry *Aphanizomenon flos-aquae* extract) 0.5-5 %
■ Perfume 0.3%

9. A composition for washing and taking care of hair,
**characterized in that** it comprises:
■ Texapon® 10.00%
■ Demineralized water qs100%
■ Sequestrene® 0.05%
■ Tego betain® 10.00%
■ Empilan® 4.00%
■ Hydralphatine 3P 3.00%
■ Antimicrobial preservative 0.50%
■ Glutamate 15.00%
■ Oramix® 6.00%
■ Simulsol® 1.00%
■ Demineralized water 5.00%
■ Acrysol® 6.00%
■ *Aphanizomenon flos-aquae* extract
in solution in sorbitol and water
(i.e. 1-5% of dry *Aphanizomenon flos-aquae* extract) 0.5-5 %

10. An antiwrinkle composition,
**characterized in that** it comprises:
■ Demineralized water qs100%
■ Sepigel® 1.0%
■ Emulium® 3.0%
■ Amerchol® 4.0%
■ Crodamol® 8.0%
■ Abil® 5.0%
■ Antimicrobial preservative 0.3%
■ Fluidamid® DF15 3.0%
■ *Aphanizomenon flos-aquae* extract
in solution in sorbitol and water
(i.e. 1-5% of dry *Aphanizomenon flos-aquae* extract) 0.5-5 %

11. A treatment mask composition for dried hair **characterized in that** it comprises:
■ Cetearyl glucoside 7%
■ Coco betaine 0.5%
■ Shea butter 4%
■ Beeswax 2%
■ Dimethicone 5%
■ Demineralized water qs100%
■ Decyl glucoside 1%
■ Perfume 0.5%
■ Antimicrobial preservative 0.5%
■ *Aphanizomenonflos-aquae* extract in solution in sorbitol and water
(i.e. 1-5% of dry *Aphanizomenon flos-aquae* extract) 0.5-5 %

12. A night cream,
**characterized in that** it comprises:
■ Cetearyl glucoside 6%
■ Vegetable oils 20%
■ DL-alpha tocopherol 0.05%
■ Dimineralized water qs100%
■ Antimicrobial preservative 0.5%
■ Perfume 0.3%
■ *Aphanizomenonflos-aquae* extract
in solution in sorbitol and water
(i.e. 1-5% of dry *Aphanizomenon flos-aquae* extract) 0.5-5%

13. A method for preparing a composition according to claim 1,
**characterized in that** it comprises the following steps:
- at least one maceration at a temperature from 25 to 50°C and, preferably 35°C, of dried *Aphanizomenon flos-aquae* blue algae in the presence of enzymes such as cellulases, pectinases, and glucanases, for a time from ten minutes to ten hours, and preferably for four hours under stirring,
- a liquid/solid separation by centrifugation,
- a liquid/liquid separation by a membrane filtration method,
- drying and/or dilution in a solution containing specific adjuvants for example sorbitol,
- optional specific separation of the different thereby extracted constituents, for example by chromatography, the different substances obtained may be used alone or as a mixture.

14. The method according to claim 13,
**characterized in that** the drying step is a standard (heat) drying step or a drying step by nebulization or freeze-drying.

15. The method according to claim 13,
**characterized in that** the aforesaid extract is dissolved in an aqueous solution such as a water/sorbitol mixture.

## Patentansprüche

1. Zur örtlichen Anwendung verwendbare Zusammensetzung,
**dadurch gekennzeichnet, dass** sie mindestens einen Extrakt aus Aphanizomenon flos-aquae in einer Konzentration enthält, die zwischen 0,01 und 10 % in der Trockensubstanz des Gesamtgewichts der Zusammensetzung liegt.

2. Zusammensetzung nach Anspruch 1,
**dadurch gekennzeichnet, dass** sie in Form einfacher oder mehrfacher Emulsionen wie einer Wasser/Öl- oder Öl/Wasser-Emulsion oder auch einer dreiphasigen Emulsion und/oder eines Gels oder einer wässrigen oder hydro-alkoholischen Lösung vorliegt.

3. Zusammensetzung nach Anspruch 1,
**dadurch gekennzeichnet, dass** sie in Form eines vektorisierten Systems zur kontrollierten oder modulierten Freisetzung vorliegt.

4. Verwendung einer Zusammensetzung nach Anspruch 1 für die Herstellung von Produkten zur Behandlung der oberen Schichten der Epidermis und/oder des Haares.

5. Verwendung einer Zusammensetzung nach einem der Ansprüche 1 bis 4 für die Herstellung eines kosmetischen Produktes.

6. Verwendung einer Zusammensetzung nach einem der Ansprüche 1 bis 4 für die Herstellung einer dermatologischen Zusammensetzung.

7. Zusammensetzung zur Pflege nach Sonnenbestrahlung,
**dadurch gekennzeichnet, dass** sie enthält:
• entmineralisiertes Wasser qsp* 100 %
• Sequestrene ® NA4/Celon ® E/Trilon ® B 0,01 %
• Nipagin ® M/POB Methyl 0,05 %
• Carbopol ® 940 15,00 %
• Triethanolamin 0,2 %
• antimikrobisches Konservierungsmittel 0,5 bis 1 %
• Silikon 1 bis 2 %
• Parfüm 0,15 %
• Extrakt aus Aphanizomenon flos-aquae
in einer Lösung aus Sorbit und Wasser (etwa 1 bis 5 % Trockenextrakt aus Aphanizomenon flos-aquae) 0,5 bis 5 %

8. Zusammensetzung zur Anti-Aging-Pflege,
**dadurch gekennzeichnet, dass** sie enthält:
• Emulium ® 4,0 %
• Amerchol ® 6,0 %
• Amerlate ® 2,0 %
• Vegetol ® öliges Calendula 2,0 %
• LNST ® 98 1 %
• entmineralisiertes Wasser qsp* 100 %
• Carbopol ® 10 %
• Abil ® 6,0 %
• entmineralisiertes Wasser 5,0 %
• Triethanolamin 0,2 %
• Antimikrobisches Konservierungsmittel 0,5 %
• Natürliches Glyzerin 4,0 %
• Fluidamid ® DF 125 4,0 %
• Extrakt aus Aphanizomenon flos-aquae
in einer Lösung aus Sorbit und Wasser (etwa 1 bis 5 % Trockenextrakt aus Aphanizomenon flos-aquae) 0,5 bis 5 %
• Parfüm 0,3 %

9. Zusammensetzung zum Waschen und Pflegen der Haare,
**dadurch gekennzeichnet, dass** sie enthält:
• Texapon ® 10,00 %
• entmineralisiertes Wasser qsp* 100 %
• Sequestrene ® 0,05 %
• Tegobetain ® 10,00 %
• Empilan ® 4,00 %
• Hydralphatin 3P 3,00 %
• antimikrobisches Konservierungsmittel 0,50 %
• Glutamat 15,00 %
• Oramix ® 6,00 %
• Simulsol ® 1,00 %
• entmineralisiertes Wasser 5,00 %
• Acrysol ® 6,00 %
• Extrakt aus Aphanizomenon flos-aquae
in einer Lösung aus Sorbit und Wasser (etwa 1 bis 5 % Trockenextrakt aus Aphanizomenon flos-aquae) 0,5 bis 5 %

10. Zusammensetzung zur Antifalten-Pflege,
**dadurch gekennzeichnet, dass** sie enthält:
• entmineralisiertes Wasser qsp* 100 %
• Sepigel ® 1,0 %
• Emulium ® 3,0 %
• Amerchol ® 4,0 %
• Crodamol ® 8,0 %
• Abil ® 5,0 %
• antimikrobisches Konservierungsmittel 0,3 %
• Fluidamid ® DF15 3,0 %
• Extrakt aus Aphanizomenon flos-aquae
in einer Lösung aus Sorbit und Wasser (etwa 1 bis 5 % Trockenextrakt aus Aphazinomenon flos-aquae) 0,5 bis 5 %

11. Zusammensetzung einer Maske zur Behandlung ausgetrockneter Haare,
**dadurch gekennzeichnet, dass** sie enthält:
• Cetaryl-Glykosid 7 %
• Kokos-Betain 0,5 %
• Karite-Butter 4 %
• Bienenwachs 2 %
• Dimeticon 5 %
• entmineralisiertes Wasser qsp* 100 %
• Decyl-Glykosid 1 %
• Parfüm 0,5 %
• antimikrobisches Konservierungsmittel 0,5 %
• Extrakt aus Aphanizomenon flos-aquae
in einer Lösung aus Sorbit und Wasser (etwa 1 bis 5 % Trockenextrakt aus Aphazinomenon flos-aquae) 0,5 bis 5 %

12. Nachtcreme,
**dadurch gekennzeichnet, dass** sie enthält:
• Cetaryl-Glykosid 6 %
• Pflanzenöle 20 %
• DL-alpha-tocopherol 0,05 %
• entmineralisiertes Wasser qsp* 100 %
• antimikrobisches Konservierungsmittel 0,5 %
• Parfüm 3 %
• Extrakt aus Aphanizomenon flos-aquae
in einer Lösung aus Sorbit und Wasser (etwa 1 bis 5 % Trockenextrakt aus Aphazinomenon flos-aquae) 0,5 bis 5 %

13. Verfahren zur Zubereitung einer Zusammensetzung nach Anspruch 1,
**dadurch gekennzeichnet, dass** es folgende Schritte aufweist:
- mindestens eine Mazeration bei einer Temperatur von 25 bis 50 °C, vorzugsweise 35 °C, von getrockneten Blaualgen Aphanizomenon flos-aquae unter Rühren in Gegenwart von Enzymen wie Cellulasen, Pectinasen und Glucanasen während eines Zeitraums von zehn Minuten bis zehn Stunden, vorzugsweise vier Stunden;
- eine Flüssig/Fest-Abtrennung durch Zentrifugieren,
- eine Flüssig/Fest-Abtrennung durch ein Membran-Filterverfahren,
- eine Trocknung und/oder Verdünnung in einer spezifische Zusatzmittel, beispielsweise Sorbit, enthaltenden Lösung,
- eine eventuelle spezifische Abtrennung von verschiedenen Bestandteilen wie Extrakten beispielsweise durch Chromatographie, wobei die erhaltenen unterschiedlichen Substanzen allein oder in Mischung verwendet werden können.

14. Verfahren nach Anspruch 13,
**dadurch gekennzeichnet, dass** der Schritt der Trocknung eine klassische (Wärme-) Trocknung oder eine Trocknung durch Zerstäubung oder Lycophilisation ist.

15. Verfahren nach Anspruch 13,
**dadurch gekennzeichnet, dass** der besagte Extrakt in einer wässrigen Lösung wie einer Wasser/Sorbit-Mischung aufgelöst ist.
